# EUROPEAN PATENT SPECIFICATION

(11) **EP 0 947 197 B1**
(45) Date of publication and mention of the grant of the patent: **21.04.2004**
(21) Application number: 99301209.5
(22) Date of filing: 18.02.1999
(51) Int. Cl.: A61K 31/575, A61K 9/14, A61K 9/16

(54) **Method for producing water dispersible sterol formulations**
Verfahren zur Herstellung wasserdispergierbare Sterol Formulierungen
Procédé pour la production de formulations contenant du stérol dispersible dans l'eau

(30) Priority: 19.02.1998 US 25952; 04.11.1998 US 185788
(43) Date of publication of application: 06.10.1999
(73) Proprietor: McNEIL-PPC, INC., Skillman, New Jersey 08558 (US)
(72) Inventor: Burruano, Brid, King of Prussia, PA 19406 (US); Hoy, Michael R., Sellersville, PA 18960 (US); Bruce, Richard D., Rydal, PA 19046 (US); Higgins, John D., Ft. Washington, PA 13034 (US)
(74) Representative: Mercer, Christopher Paul

(56) References cited:
- EP-A- 0 357 967
- US-A- 3 881 005
- US-A- 4 195 084
- SJOSTROM B ET AL: "A METHOD FOR THE PREPARATION OF SUBMICRON PARTICLES OF SPARINGLY WATER-SOLUBLE DRUGS BY PRECIPITATION IN OIL-IN-WATER EMULSIONS II: INFLUENCE OF THE EMULSIFIER, THE SOLVENT, AND THE DRUG SUBSTANCE" JOURNAL OF PHARMACEUTICAL SCIENCES, vol. 82, no. 6, 1 June 1993, pages 584-589, XP000367863

## Description

### BACKGROUND OF THE INVENTION

The present invention relates to a method of producing spray dried aqueous-dispersible sterol formulations, in particular a method for producing dispersible β-sitosterol via a spray drying process.

As disclosed in U.S. Patent Nos. 5,502,045, 5,578,334 and 5,244,877, it is known that consumption of β-sitosterol is known to reduce cholesterol levels in the blood stream. Presently, β-sitosterol is incorporated in foods as an ingredient of the food while it is being prepared. While this is effective in producing foods with beneficial effects, the consumer is limited to those foods in which the manufacturers have decided to incorporate the β-sitosterol.

It would be highly desirable to provide β-sitosterol in a convenient ready to consume form which consumers could apply to food just prior to eating. A particularly convenient form would be a single serving packet of β-sitosterol similar to those that are currently available for artificial sweeteners. The difficulty in providing β-sitosterol in this form is that it is difficult to separate the active ingredient from other sterols, namely stigmasterol, campesterol and the like.

Attempts at solving this problem are disclosed in US Patent 3,881,005 and US 4,195,084 in which water dispersible sitosterols are formed by mixing with an excipient and a suitable surfactant, While this disclosure produces a water dispersible sitosterol, it would be highly advantageous to improve the water dispersibility of the β-sitosterol, since this is believed to be the more effective form as a cholesterol lowering agent.

### SUMMARY OF THE INVENTION

A primary object of the present invention is to provide a method for preparing a stable spray dried powder matrix that has self-emulsifying character upon addition to aqueous media. According to the present invention there is provided a process as defined in claim 1. The process of the present invention is performed in the absence of deaeration and homogenization steps.

### DETAILED DESCRIPTION OF THE INVENTION

β-sitosterols are typically derived from wood or agricultural sources, such as soy based mixtures. In addition to β-sitosterol, as used throughout this application, β-sitosterol is also understood to include the esters of β-sitosterols, as well as stanol and stanol ester derivatives which are the reduced derivatives of the sterols. These derivatives are well known in the art and include patents US 5,244,887; US 5,502,045 and US 5,698,527. The β-sitosterols produced by the present invention are water dispersible. As used herein, water dispersible is understood to mean that when the β-sitosterol spray dried formulation is placed in water, at least 200 mg formula/ml water will disperse with mild agitation. Those with skill in the art will appreciate that ordinarily β-sitosterols are hydrophobic materials, and upon the addition of the β-sitosterol to water, the β-sitosterol will float on top of the water and will not become dispersed.

The present invention is also applicable to another class of cholesterol-lowering compounds, oryzanol and its esters. These materials are also known in the art as well as the esters of the oryzanol compound, see for example, U.S. Patent No. 5,514,398 and PCT WO 98/01519. The present invention also provides the oryzanol, esters of oryzanol and other related compounds in a more dispersible form. Although the remaining specification will refer to β-sitosterols, the present invention is equally applicable to said oryzanol and related compounds.

In order to be most effective when ingested, the particle size of the β-sitosterol should be in the range of from 10 to 40 µm. More preferably the particle size should from about 20 to 35 µm. Any grinding technique known in the art may be used to grind the β-sitosterol. Suitable methods include pulverizing, rotary hammermill, air milling and the like of which air milling is most preferred. Smaller particles sizes are preferred in that the resulting β-sitosterol product is more readily exposed to bile salts in the digestive tract. The handling properties of the smaller particle size product are less desirable, resulting in higher angle of rupture, higher angle of repose and compressibility. The handling of the water-dispersible β-sitosterol product can be improved with increased particle size; however, this is believed to be detrimental to the efficacy of the β-sitosterol in reducing serum cholesterol.

In order to form the water dispersible β-sitosterols appropriate surfactants are required. The present invention employs a dual surfactant system. One surfactant in the system is monofunctional, while the second surfactant is polyfunctional. The monofunctional surfactants tend to be more hydrophobic, whereas the polyfunctional surfactants tend to be hydrophilic. The two-surfactant system employed in this invention creates a mixed micelle system that results in the water-dispersible product. As used herein the monofunctional surfactant is polyoxyethylene sorbitan monopalmitate which has the ability to bond to the β-sitosterol. The polyfunctional surfactant is sorbitan monooleate which has the ability to bond to the β-sitosterol as well as to the other surfactant.

A suitable monofunctional surfactant is, with HLB values provided in brackets, [ ] polyoxyethylene (20) sorbitan monopalmitate [15.6]. As is appreciated by those with skill in the art, the HLB value for a surfactant is an expression of its Hydrophile-Lipophile balance, i.e., the balance of the size and strength of the hydrophilic (polar) and lipophilic (non-polar) groups of the surfactant.

The level of monofunctional surfactant is typically from about 1 to about 10 weight percent based upon the final dried weight of the β-sitosterol product, preferably from about 1.5 to about 4, and most preferably about 2.0 to about 2.5 weight percent. The level of polyfunctional surfactant is typically from about 0.5 to about 10 weight percent based upon the final dried weight of the β-sitosterol product, preferably from about 2 to about 4, and most preferably about 2.0 to about 2.5 weight percent. TWEEN 40 is the preferred monfunctional surfactant and SPAN 80 is the preferred polyfunctional surfactant. Suitable ratios of mofunctional / polyfunctional surfactants which form the mixed micelle include from about 1:6 to about 1.5:1, preferably from about 1:4 to about 1.3:1, most preferably about 1:1 ratio. The level of surfactant employed ranges from about 0.5 to about 8 percent by weight total surfactant system, preferably 1 to about 6, most preferably from about 3 to about 4 percent by weight.

In a preferred embodiment, in addition to the surfactant, other excipients, tableting aids etc. are added to the formulation as the suspension is formed, prior to the spray drying process. This conveniently incorporates tableting aids and other necessary ingredients thereby eliminating or reducing unit-manufacturing steps. If desired, ingredients can also be added to the β-sitosterol after spray drying.

For example, lubricants, glidants, carriers, sweeteners, disintegrants, preservatives and other ingredients may be added to the suspension in the amount of from about 5 to about 40 weight percent, typically from about 10 to about 30 percent and most preferably to about 20 to about 25 percent. Suitable ingredients include binders are acacia mucilage, starch mucilage pregelatinised starch, sodium alignate, hydroxypropylmethyl cellulose (HPMC), starch paste, polyvinylpyrrolidone, carboxymethylcellulose, dextrin, ethyl cellulose, polyethylene glycol, guar gum, zein, hydroxyethyl cellulose, hydroxypropyl cellulose, methyl cellulose, polymethacrylates, and carboxymethylcellulose.

Disintegrating agents include microcystalline cellulose (e.g. Avicel R), sodium carboxymethyl cellulose (e.g. Nymcel R), modified cellulose gum (e.g. Ac-Di-Sol R), crosslinked providone, alginic acid and alginates, pregelatinised starch, sodium starch glycollate (e.g. Explotab R, Primojel R), modified corn starch (e.g. starch 1500R), starch (e.g. potato/maize starch), and ion exchange resin such as polacrin potassium (e.g. Amberlite IRP-88).

Examples of water-soluble fillers are: soluble lactose, compressible sugar, confectioners sugar, dextrose, mannitol, sodium chloride, sorbitol, xylitol. Examples of water-insoluble fillers are: calcium carbonate, magnesium carbonate, calcium phosphate (e.g. di and tri basic calcium phosphate), calcium sulphate, kaolin, microcystalline cellulose, powdered cellulose, pregelatinized starch, barium sulphate, magnesium trisilcate, aluminum hydroxide.

Generally lubricants are used in as low an amount as possible. Examples of lubricants include: stearates (e.g. magnesium or calcium stearate), talc, polyethylene glycol, liquid paraffin, sodium lauryl sulphate, magnesium lauryl sulphate, colloidal silicone dioxide, palmitostearate, stearic acid, zinc stearate, hydrogenated vegetable oil.

Glidants including talc, starch, magnesium stearate, silica derivatives, such as colloidal silica (e.g. AEROSIL) pyrogenic silica, hydrated sodium silicoaluminate, colloidal silicon dioxide.

Flavoring agents including orange, cherry, and strawberry, raspberry, grape and passion fruit.

Sweetening agents, include for example, sodium saccharin, aspartame, confectioners sugar, sorbitol, xylitol and mixtures thereof.

The β-sitosterol and the other ingredients in the suspension should be uniformly mixed. Preferably the suspension is mixed by agitation, preferably through the use of a high-speed mixer. The particle size of the micelles in the suspension formed are from about 50 to about 400 µm preferably from about 100 to about 300 µm and most preferably from about 150 to about 250 µm in size. The size of the micelles formed in the suspension may be measured through the use of a Turbimeter. The greater turbidity, the larger the micelle formation. It is expected that greater turbidty, i.e., larger micelles provides a more effective form of the β-sitosterol for reducing cholesterol when consumed. Preferred turbidity levels are greater than about 2000, preferably greater than 2500 and most preferably greater than 3000 Nephelometric Turbidity Units (NTU). As used herein turbidity is understood to be the same as defined by the United States Pharmacopeia, the light scattering effect of suspended particles and turbidity as the measure of the decrease in the incident beam intensity per unit length of a given suspension. The range of turbidty values is from 0 to 20,000 NTU. As a point of reference the turbidity of water is zero. The turbidity of the samples was measured at room temperature.

After the suspension with the proper particle size is formed the suspension is dried. Suitable drying methods include freeze drying, rotary, vacuum and spray drying, of which spray drying is preferred. The final moisture content of the dried β-sitosterol is preferably less than 1% by weight water. Lower moisture content generally provides improved flow characteristics.

When spray drying the suspension, it is preferable that the inlet temperature is from about 100 to 120°C, preferably from about 105 to about 115°C and most preferably from 107 to about 112°C. The outlet temperature of the spray dryer is between about 65 and 85°C and most preferably from about 73 to about 80°C.

The spray dried water-dispersible β-sitosterol formulation is then recovered. The resulting water-dispersible β-sitosterol is comprised of from greater than 50 percent by weight sterol, greater than 4 and preferable from about 5 to about 10 weight percent surfactant, based on the final dried weight of β-sitosterol formulation. In a highly preferred embodiment the β-sitosterol also includes about 5 percent starch and about 5 percent silicon dioxide.

After the β-sitosterol is removed from the dryer it is packaged in any suitable size as may be required. The form in which the β-sitosterol is consumed varies depending on the preference of the consumer. Suitable forms include tablets, chewable dosages, in the preparation of food and beverages as well as applied to prepared beverages and foodstuffs. In a preferred embodiment, the β-sitosterol may be packaged in single serving size packets containing from about 5 to about 50 grams per packet.

The present invention provides advantages over previous disclosures that provide water-dispersible β-sitosterols in that several costly and time consuming process steps are eliminated. Prior disclosures required both a homogenization and deaeration step in order to produce the water-dispersible β-sitosterol. The present invention provides the water-dispersible β-sitosterol through the use of the selection of advantageous combinations of surfactants. The invention will now be illustrated by the following examples. In the examples the starch was ground to a particle size of approximately 10 µm. In these examples it is understood that unless noted otherwise, all parts are weight percent. The following raw materials are available from the following suppliers.
CAB O SIL colloidal silicon dioxide, Degussa Corp.
AEROSIL A200 colloidal silicon dioxide, Cabot Corp.
EM Compress dibasic calcium phosphate dihydrate, Edward Mendall Compress Co., Inc.
M100 maltrodextrin (dextrose equivalent of about 10) Grain Process Corp.
Pluronic L-44 a polyethylene-propylene glycol copolymer, BASF Corp.
SPAN 80 sorbitan monooleate, ICI Americas, Inc.
Starch: Starch NF-, National Starch and Chemicals Inc.
Sterols: Generol 122N available from Henkel Company, Ambler, PA.
TWEEN 40 polyoxyethylene 20 sorbitan monopalmitate, ICI Americas Inc.
TWEEN 60 polyoxyethylene 20 sorbitan monostearate, ICI Americas Inc.

### EXAMPLE 1: (Comparative Example)

This example discloses a formulation for spray dried material containing approx. 75% sterols (based on dry weight). Any polyoxyethylene sorbitan fatty acid ester can be incorporated in the place of TWEEN 60.

| Component: | Amount (gm): |
|---|---|
| TWEEN 60 | 30 |
| Maltodextrin - Maltrin M100 | 240 |
| Aerosil A200 | 22 |
| Starch NF | 75 |
| Phytosterols | 1,120 |
| Water | 10,000 |

The sample is prepared as follows: the TWEEN 60 and 500 gm water of water was added and the mixture was stirred on a hot plate set at 60°C until uniform. The solution was transferred into a larger container, rinsing with water. An additional remaining 9,500gm of water was added. The starch, Maltrin M100, Aerosil 200 and the sterols were weighed out and added to the solution. The resulting solution was mixed with high shear mixer for approximately 1 hour. The suspension was spray dried immediately afterwards.

The turbidity of 100 mg of the resulting spray dry powder in 25 ml of water was approximately 300 NTU.

### EXAMPLE 2: (Comparative Example)

The following example outlines a spray dry formulation containing approx. 75% sterols (based on dry weight).

| Component: | Amount (gm): |
|---|---|
| Docusate Sodium | 40 |
| Maltodextrin - Maltrin M100 | 240 |
| Aerosil A200 | 22 |
| Starch NF | 75 |
| Phytosterols | 1,120 |
| Water | 10,000 |

The sample is prepared as follows: the docusate sodium was weighed into a beaker, 500 gm water of water was added and the mixture was stirred on a hot plate set at 60°C until uniform. The solution was transferred into a larger container, rinsing with water. An additional remaining 9,500 gm of water was added. The starch, Maltrin M100, Aerosil 200 and the sterols were weighed out and added to the solution. The resulting solution was mixed with high shear mixer for approximately 1 hour. The suspension was spray dried immediately afterwards.

The turbidity of the 100 mg of the resulting spray dried powder in 25 ml of water was approximately 2400 NTU.

### EXAMPLE 3: (Comparative Example)

The following example outlines a spray dry formulation containing approx. 75% sterols (based on dry weight). Any poloxamer can be incorporated in place of Pluronic L-44.

| Component: | Amount (gm): |
|---|---|
| Pluronic L-44 | 80 |
| Maltodextrin - Maltrin M100 | 240 |
| Aerosil A200 | 22 |
| Starch NF | 75 |
| Phytosterols | 1,120 |
| Water | 10,000 |

The sample is prepared as follows: the Pluronic L-44 was weighed into a beaker, 500gm water of water was added and the mixture was stirred on a hot plate set at 60°C until uniform. The solution was transferred into a larger container, rinsing with water. An additional remaining 9,500gm of water was added. The starch, Maltrin M100, Aerosil 200 and the sterols were weighed out and added to the solution. The resulting solution was mixed with high shear mixer for approximately 1 hour. The suspension was spray dried immediately afterwards.

The Turbidity of the 100 mg of the resulting spray dried powder in 25 ml of water was approximately 2600 NTU.

| Component: | Amount (gm): |
|---|---|
| TWEEN 40 | 40 |
| SPAN 80 | 40 |
| Maltodextrin - Maltrin M100 | 240 |
| Aerosil A200 | 22 |
| Starch NF | 75 |
| Phytosterols | 1,120 |
| Water | 10,000 |

The sample was prepared as follows: the Tween and Span were weighed into a beaker, 500gm water of water was added and the mixture was stirred on a hot plate set at 60°C until uniform. The solution was transferred into a larger container, rinsing with water. An additional remaining 9,500gm of water was added. The starch, Maltrin M100, Aerosil 200 and the sterols were weighed out and added to the solution. The resulting solution was mixed with high shear mixer for approximately 1 hour. The suspension was spray dried immediately afterwards.

The turbidity of the 100 mg of the resulting spray dried powder in 25 ml of water was approximately 3500 NTU.

### EXAMPLE 5

Three separate spray-drying experiments were conducted using 3 different phytoactive compounds. The phytoactive compounds were: β-sitisterol, β-sitostanol and oryzanol A. The remaining formulation including the phytoactive compounds included:

| COMPONENT | FORMULA % DRY BASIS | FORMULA % WET BASIS |
|---|---|---|
| TWEEN 40 | 1.98 | 0.34 |
| SPAN 80 | 1.98 | 0.34 |
| Maltodextrin (MALTRIN M100) | 15.82 | 2.74 |
| AEROSIL 200 | 1.45 | 0.25 |
| Starch N.F. | 4.94 | 0.86 |
| Phytoactive compound | 73.83 | 12.81 |
| Purified water | Not applicable | 82.65 |
| Dry Basis Total | 100.0 | Not applicable |
| Wet Basis Total | Not applicable | 100.00 |

The turbidity of 100 mg of the resulting spray dried powders in 25 ml of water was as follows:

| Phytoactive Compound | Turbidity |
|---|---|
| β-sitosterol | 3155 NTU |
| β-sitostanol | 4260 NTU |
| γ-Oryzanol | 2063 NTU |

### EXAMPLE 6

Spray dried material prepared according to the method described in example 5 was combined with inactive ingredients to produce tablets according to the formula and process described below:

| Material | Quantity (mg/tablet) |
|---|---|
| Stanol-74 SD granule (73.83% active) | 677.2 |
| Stearic Acid | 4.4 |
| Croscarmelose Sodium | 30.8 |
| Colloidal Silicon Dioxide | 10.5 |
| Microcrystalline Cellulose | 40.0 |
| Tablet weight | 762.9 |

a. All ingredients were combined in a plastic bag, and blended for 5 minutes.
b. The blend was compressed into tablets using a Carver press at 900 pounds force (approximately 4032 Newtons) for 3 seconds using caplet shaped tooling 750 X 350X 60 1and tooling (measurements in thousanths of an inch) (or approximately 1.9 x .89 x 0.15 centimeters). Tablets were compressed to the following targets:
   Average Weight (mg): 763
   Thickness (mm): 6.36

### Test Results:

| | |
|---|---|
| Hardness (average) | 11 kp |
| Disintegration* time | 20 minutes |

| | |
|---|---|
| * Apparatus: USP 23 <701> p.1791 with 900 milliliters of deionized water at 37°C. | |

## Claims

1. A process for preparing water-dispersible β-sitosterol or oryzanol formulation comprising:
a) providing an aqueous medium;
b) admixing to the aqueous medium from 1 to 10 weight percent of a monofunctional surfactant and from 0.5 to 10 weight percent of a polyfunctional surfactant to form a water surfactant mixture, said percentages being based upon the final dried weight of the formulation;
c) admixing β-sitosterol or oryzanol to the water surfactant mixture to form a β-sitosterol or oryzanol suspension;
d) drying the β-sitosterol or oryzanol suspension to recover a water-dispersible β-sitosterol or oryzanol;
wherein the above process is performed in the absence of both deaeration and homogenization steps, and
wherein the β-sitosterol or oryzanol suspension has a turbidity of greater than 2000 NTU, and wherein the monofunctional surfactant is polyoxyethylene sorbitan monopalmitate and the polyfunctional surfactant is sorbitan monooleate.

2. The process of claim 1 wherein the monofunctional surfactant is used from 2 to 2.5 weight percent and the polyfunctional surfactant is used from 2 to 2.5 weight percent.

3. The process of claim 2 wherein the weight ratio of the monofunctional surfactant to the polyfunctional surfactant is about 1:1.

4. The process of any one of claims 1 to 3 wherein the β-sitosterol or oryzanol suspension is formed through the use of a high-speed mixer.

5. The process of claim 4 wherein the β-sitosterol or oryzanol is ground prior to the formation of the β-sitosterol or oryzanol suspension.

6. A single serving package providing from 5 to 50 grams of water-dispersible β-sitosterol or oryzanol formulation produced by the process of any one of claims 1 to 5.

7. A tablet comprising the water-dispersible β-sitosterol or oryzanol formulation produced by the process of any one of claims 1 to 5.

## Patentansprüche

1. Verfahren zur Herstellung einer Wasser-dispergierbaren β-Sitosterol- oder Oryzanol-Formulierung, umfassend:
a) zur Verfügung stellen eines wäßrigen Mediums;
b) Hinzumischen zu dem wäßrigen Medium von 1 bis 10 Gewichtsprozent eines monofunktionellen Oberflächen-aktiven Mittels und von 0,5 bis 10 Gewichtsprozent eines polyfunktionellen Oberflächen-aktiven Mittels, um ein Wasser-Oberflächen-aktives Mittel-Gemisch zu bilden, wobei die Prozentsätze auf dem finalen Trockengewicht der Formulierung basieren,
c) Hinzumischen von β-Sitosterol oder Oryzanol zu dem Wasser-Oberflächenaktiven Mittel-Gemisch, um eine β-Sitosterol- oder Oryzanol-Suspension zu bilden;
d) Trocknen der β-Sitosterol- oder Oryzanol-Suspension, um ein Wasserdispergierbares β-Sitosterol oder Oryzanol zu erhalten;
wobei das oben genannte Verfahren in der Abwesenheit von sowohl Entlüftungs- und Homogenisierungsschritten durchgeführt wird, und
worin die β-Sitosterol- oder Oryzanol-Suspension eine Trübung von höher als 2000 NTU aufweist, und wobei das monofunktionelle Oberflächen-aktive Mittel Polyoxyethylensorbitanmonopalmitat ist und das polyfunktionelle Oberflächen-aktive Mittel Sorbitanmonooleat ist.

2. Verfahren nach Anspruch 1, wobei das monofunktionelle Oberflächen-aktive Mittel in von 2 bis 2,5 Gewichtsprozent und das polyfunktionelle Oberflächen-aktive Mittel in von 2 bis 2,5 Gewichtsprozent verwendet wird.

3. Verfahren nach Anspruch 2, wobei das Gewichtsverhältnis des monofunktionellen Oberflächen-aktiven Mittels zu dem polyfunktionellen Oberflächen-aktiven Mittel ungefähr 1:1 ist.

4. Verfahren nach einem der Ansprüche 1 bis 3, wobei die β-Sitosterol- oder Oryzanol-Suspension durch die Verwendung eines Hochgeschwindigkeitsmischers gebildet wird.

5. Verfahren nach Anspruch 4, wobei das β-Sitosterol oder Oryzanol vor der Bildung der β-Sitosterol- oder Oryzanol-Suspension gemahlen wird.

6. Einzeln portionierte Verpackung, die von 5 bis 50 Gramm einer durch das Verfahren nach einem der Ansprüche 1 bis 5 hergestellten Wasser-dispergierbaren β-Sitosteroloder Oryzanol-Formulierung zur Verfügung stellt.

7. Tablette, umfassend die durch das Verfahren nach einem der Ansprüche 1 bis 5 hergestellte Wasser-dispergierbare β-Sitosterol- oder Oryzanol-Formulierung.

## Revendications

1. Procédé de préparation de la formulation de β-sitostérol ou d'oryzanol dispersible dans l'eau comprenant :
a) la mise à disposition d'un milieu aqueux ;
b) l'addition de 1 à 10% en poids d'un agent tensioactif monofonctionnel et de 0,5 à 10% en poids d'un agent tensioactif polyfonctionnel au milieu aqueux pour former un mélange d'agents tensioactifs et d'eau, lesdits pourcentages étant basés sur le poids sec final de la formulation ;
c) l'addition de β-sitostérol ou d'oryzanol au mélange d'agent tensioactif et d'eau pour former une suspension de β-sitostérol ou d'oryzanol ;
d) le séchage de la suspension de β-sitostérol ou d'oryzanol pour récupérer du β-sitostérol ou de l'oryzanol dispersible dans l'eau ;
dans lequel le procédé ci-dessus est effectué en l'absence des étapes à la fois d'évacuation de l'air et d'homogénéisation, et
dans lequel la suspension de β-sitostérol ou d'oryzanol a une turbidité plus grande que 2000 NTU, et
dans lequel l'agent tensioactif monofonctionnel est le monopalmitate de polyoxyéthylène sorbitane et l'agent tensioactif polyfonctionnel est le monooléate de sorbitane.

2. Procédé de la revendication 1 dans lequel l'agent tensioactif monofonctionnel est utilisé à hauteur de 2 à 2,5% en poids et l'agent tensioactif polyfonctionnel est utilisé à hauteur de 2 à 2,5% en poids.

3. Procédé de la revendication 2 dans lequel le rapport en poids de l'agent tensioactif monofonctionnel par rapport à l'agent tensioactif polyfonctionnel est d'environ 1:1.

4. Procédé de l'une quelconque des revendications 1 à 3 dans lequel la suspension de β-sitostérol ou d'oryzanol est formée par l'utilisation d'un mélangeur à grande vitesse.

5. Procédé de la revendication 4 dans lequel le β-sitostérol ou l'oryzanol est broyé avant la formation de la suspension de β-sitostérol ou d'oryzanol.

6. Conditionnement en portion individuelle proposant de 5 à 50 grammes de formulation de β-sitostérol ou d'oryzanol dispersible dans l'eau produite par le procédé de l'une quelconque des revendications 1 à 5.

7. Pastille comprenant la formulation de β-sitostérol ou d'oryzanol dispersible dans l'eau produite par le procédé de l'une quelconque des revendications 1 à 5.
